Europäisches Patentamt

European Patent Office

Office européen des brevets

(11) Veröffentlichungsnummer: **0 158 362**
**B1**

(12) # EUROPÄISCHE PATENTSCHRIFT

(45) Veröffentlichungstag der Patentschrift:
13.07.88

(51) Int. Cl.⁴: **C 07 D 251/28**

(21) Anmeldenummer: **85104433.9**

(22) Anmeldetag: **11.04.85**

(54) Verfahren zur Gewinnung von festem Cyanurchlorid.

(30) Priorität: **13.04.84 DE 3414097**

(43) Veröffentlichungstag der Anmeldung:
**16.10.85 Patentblatt 85/42**

(45) Bekanntmachung des Hinweises auf die Patenterteilung:
**13.07.88 Patentblatt 88/28**

(84) Benannte Vertragsstaaten:
**BE CH DE IT LI**

(56) Entgegenhaltungen:
**AT - B - 265 296**
**CH - A - 386 437**
**DE - B - 2 843 379**
**US - A - 3 141 882**

**Die Akte enthält technische Angaben, die nach dem
Eingang der Anmeldung eingereicht wurden und die
nicht in dieser Patentschrift enthalten sind.**

(73) Patentinhaber: **SKW Trostberg Aktiengesellschaft,
Dr.-Albert-Frank-Strasse 32 Postfach 1150/1160,
D-8223 Trostberg (DE)**

(72) Erfinder: **Klima, Hubertus, Siedlerstrasse 3,
D-8221 Tacherting (DE)**
Erfinder: **Jekat, Herbert, Dr., Breslauer Strasse 73,
D-7860 Schopfheim (DE)**

(74) Vertreter: **Huber, Bernhard, Dipl.-Chem. et al,
Patentanwälte H. Weickmann, Dr. K. Fincke F.A.
Weickmann, B. Huber Dr. H. Liska, Dr. J. Prechtel
Möhlstrasse 22 Postfach 860 820,
D-8000 München 86 (DE)**

**Beschreibung**

Gegenstand der vorliegenden Erfindung ist ein Verfahren zur Gewinnung von festem Cyanurchlorid aus dem bei der Trimerisierung von Chlorcyan anfallenden Cyanurchloriddampf.

Cyanurchlorid besitzt als technisches Zwischenprodukt für die Herstellung von Farbstoffen, Pflanzenschutzmitteln, Pharmazeutika sowie Textil- und Kautschukhilfsmitteln erhebliche technische Bedeutung und fällt nach der katalytischen Trimerisierung von Chlorcyan in gasförmiger Form zusammen mit nichtumgesetztem Chlorcyan und Chlor an. Dieses Gasgemisch wird üblicherweise in Abscheidekammern geleitet und das Cyanurchlorid an den gekühlten Wänden abgeschieden. Nachteilig bei dieser Art der Desublimation ist der Umstand, daß sich das Cyanurchlorid in Form grober Kristalle an den Wänden und Austragsvorrichtungen absetzt und somit den Wärmeübergang negativ beeinflußt. Das regelmäßige Abklopfen der Anbackungen von den Wänden führt lediglich zu einer kurzzeitigen Verbesserung der Wärmeübergangs. Weiterhin ist diese Methode wegen der zunehmenden Beschädigung des Abscheiders und der Lärmbelästigung keineswegs befriedigend, ganz abgesehen von der schlechten Qualität des auf diese Weise erhaltenen Produktes.

Gemäß der DE-AS 12 66 308 hat man versucht, dieses Problem zu lösen, indem man das Cyanurchlorid zusammen mit einer leicht verdampfenden Kühlflüssigkeit, wie z. B. Methylenchlorid oder Chloroform, versprühte. Man erhält auf diese Weise zwar ein feinverteiltes Cyanurchlorid, doch ist die Rückgewinnung der Kühlflüssigkeit technisch sehr aufwendig. Darüber hinaus kommt es sehr leicht zu Verstopfungen der Düse. Anstelle der direkten Abscheidung des Cyanurchloriddampfes wurde z. B. in DE-PS 25 37 673 und DE-PS 23 32 636 vorgeschlagen, das im Reaktionsgas enthaltene Cyanurchlorid vor der Verfestigung zu verflüssigen und anschließend zu versprühen, wobei die Abführung der Desublimationswärme geringere Probleme aufwirft und Chlor und Chlorcyan vor der Verfestigung entfernt werden können. Dieses zweistufige Abscheideverfahren ist technisch aufwendig. Diesen Nachteil weisen auch die Verfahren gemäß der deutschen Auslegeschriften 28 43 381 und 28 43 382 zur Gewinnung von festem oder flüssigem Cyanurchlorid auf, bei denen man das nach der Trimerisierung von Cyanurchlorid anfallende Reaktionsgemisch in eine Apparatekombination, bestehend aus Abtriebskolonne und Kondensator, einleitet und durch Temperaturregelung am Austritt des Kondensators das Cyanurchlorid in der Kolonne teilweise kondensiert, während der gasförmige Anteil, der am Kolonnenkopf austritt, in den üblichen Abscheidekammern desublimiert wird. Dieses Verfahren verursacht jedoch hohe Betriebs- und Investitionskosten.

Der vorliegenden Erfindung lag daher die Aufgabe zugrunde, ein Verfahren zur Gewinnung von festem Cyanurchlorid zu entwickeln, das diese Nachteile des Standes der Technik nicht aufweist und das es ohne großen technischen Aufwand ermöglicht, ein feinkörniges Cyanurchlorid mit einem engen Kornspektrum herzustellen.

Diese Aufgabe wurde gelöst durch ein Verfahren zur Gewinnung von festem Cyanurchlorid aus dem bei der Trimerisierung von Chlorcyan anfallenden Cyanurchloriddampf, das dadurch gekennzeichnet ist, daß man den Cyanurchloriddampf im oberen Teil der Abscheidekammer in das Zentrum vieler einzelner kalter Inertgasströme einleitet, wobei durch ein in die Abscheidekammer eingebautes Zentralrohr eine Vormischung von kaltem Inertgas aus dem Kühler und wärmerem Kreisgas aus der Abscheidekammer bewirkt wird, wonach eine Vermischung dieses Gemisches mit dem heißen Cyanurchloriddampf erfolgt und wobei die Inertgasströme mit einer Temperatur von 0° bis 40°C und einer Geschwindigkeit von 40 bis 80 Meter pro Sekunde in einem 50- bis 100fachen Überschuß, bezogen auf die Menge an Cyanurchloriddampf, eingeleitet werden, das feste Cyanurchlorid nach dessen Austritt aus der Abscheidekammer durch Einwirken von Zentrifugal- und Schwerkraft von Inertgasstrom abtrennt und das Inertgas mit einem Restgehalt von 15 bis 20 Gew.-% an feinstem Cyanurchlorid, bezogen auf das Gesamtgewicht des Cyanurchlorids, nach dessen Abkühlung wieder als Teilströme in die Abscheidekammer zurückführt.

Überraschenderweise wurde gefunden, daß man mit dem erfindungsgemäßen Verfahren ein sehr feinteiliges Cyanurchlorid mit sehr hoher Reinheit gewinnen kann. Darüber hinaus ist eine rasche Abscheidung des festen Cyanurchlorids möglich, ohne daß Probleme bei der Wärmeabführung durch Anbackungen an der Wand oder Verstopfungen auftreten.

Gemäß der vorliegenden Erfindung wird das bei Herstellung des Cyanurchlorids anfallende Reaktionsgemisch so in den oberen Teil einer Abscheidekammer eingeleitet, daß es sich im Zentrum vieler einzelner kalter Inertgasströme befindet und somit vom Inertgas eingehüllt wird. Auf diese Weise wird verhindert, daß der Cyanurchloriddampf mit den Wänden des Abscheiders in Berührung kommt und daß sich grobe Kristalle oder Agglomerate bilden. Die Inertgasströme haben bevorzugt eine Temperatur im Bereich von 15° bis 30°C. In diesem Temperaturbereich ist der Abschreckeffekt durch das Inertgas in jedem Fall ausreichend. Das Inertgas tritt mit in die Abscheidekammer ein, wodurch eine gute Vermischung mit dem Cyanurchloriddampf und eine rasche Abscheidung des Feststoffes erreicht wird. Die Aufspaltung des Inertgasstroms in einzelne Teilströme erfolgt durch Kühlerrohre, die konzentrisch um die Cyanurchloridzuleitung angeordnet sind.

Als Inertgas können prinzipiell alle Gase eingesetzt werden, die mit dem Cyanurchloriddampf bei den entsprechenden Temperaturen keine Reaktion eingehen. Aus wirtschaftlichen Gründen werden trockene Luft oder Stickstoff besonders bevorzugt. Nach der Abscheidung des Cyanurchlorids verlassen die feinteiligen Feststoffpartikel zusammen mit dem erwärmtem Inertgas die Abscheidekammer und werden der Einwirkung von Zentrifugal- und Schwerkraft ausgesetzt, wobei eine teilweise Trennung des Gases vom Feststoff erfolgt. Diese Trennung erfolgt vorzugsweise in einem Zyklon. Das Inertgas hat dann noch einen Restgehalt an feinstem kristallinem Cyanurchlorid. Dieser Restgehalt an feinsten Cyanurchloridteilchen ist wesentlich, da diese Teilchen zusammen mit dem Inertgas nach dessen Verdichtung und Abkühlung in einem Wärmeaustauscher wieder in die Abscheidekammer geleitet werden und dort als Kristallisationskeime wirken. Auf diese Weise wird die Desublimation des dampfförmigen Cyanurchlorids zum kontrollierten Kristallisationsvorgang. Die mitgeschleppten feinsten Partikel beeinflussen die Geschwindigkeit der Desublimation und damit die Teilchengröße des abgeschiedenen Cyanurchlorids. Mit Hilfe des erfindungsgemäßen Verfahrens ist es somit möglich, ein Cyanurchlorid herzustellen, bei dem 95% der Teilchen eine Größe von weniger als 63 µm haben und das ein Schüttgewicht von 200 bis 600 kg/m³ aufweist. Auf diese Weise kann die Korngröße des Produkts gesteuert werden, was auch über die Temperatur und Menge des Inertgases möglich ist. Besonders vorteilhaft ist es, die zirkulierenden feinsten Partikel des Cyanurchlorids auf eine Korngröße von weniger als 5 µm zu begrenzen. Dazu läßt man die Zentrifugal- und Schwerkraft so einwirken, daß alle Teilchen, die größer als 5 µm sind, als Feststoffpartikel abgeschieden werden und daß die Teilchen mit einer Korngröße von weniger als 5 µm in dem Inertgas verbleiben. Dabei ist es bevorzugt, daß die Menge der in dem Inertgas verbleibenden Teilchen 15 bis 20 Gew.-% der Gesamtmenge des Cyanurchlorids ausmacht.

Da der Inertgasstrom, von dem die Feststoffpartikel abgetrennt sind, noch geringe Verunreinigungen an Chlorcyan oder Chlor enthält, ist es erforderlich, kontinuierlich einen Teil des Inertgases vor dessen Verdichtung bzw. Kühlung abzuziehen und diesen nach den üblichen Verfahren zu reinigen.

In einer bevorzugten Ausführungsform des erfindungsgemäßen Verfahrens wird am Kopf der Abscheidekammer ein Zentralrohr eingebaut, das aus einem Mischrohr und einem Diffusor besteht. In dieses Zentralrohr wird das aus dem Kühler austretende inerte, kalte Gas eingeleitet. Es wirkt als Treibstrahl und saugt aus dem äußeren Ringspalt der Abscheidekammer eine große Menge Kreisgas höherer Temperatur an. Nach Vermischung der beiden Gasströme stellt sich eine höhere Temperatur ein als ohne diese Einrichtung. In diese Zone wird der heiße Cyanurchloriddampf eingeleitet. Der Abschreckeffekt ist dadurch gemildert. Ferner werden durch diese Einrichtung feinste Kristalle wiederholt durch die Zone des Übersättigungsabbaus geführt. Aus beidem resultiert ein etwas grobkörnigeres Produkt.

Auch bei dieser Verfahrensvariante ergeben sich die erfindungsgemäßen Vorteile des Verfahrens wie hohe Leistungsdichte, Vermeidung von Anbackungen, schnelle Abscheidegeschwindigkeit und ein enges Korngrößenspektrum.

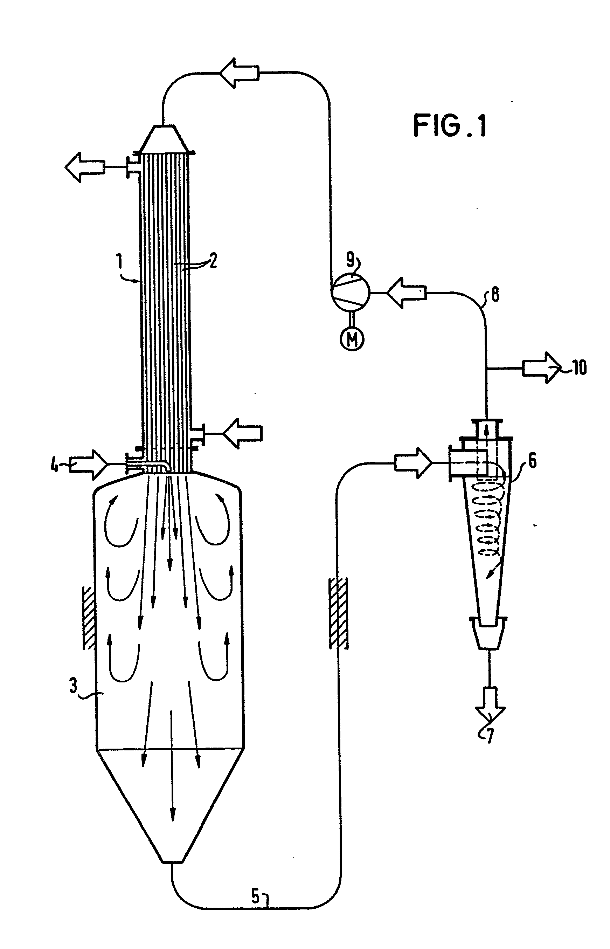

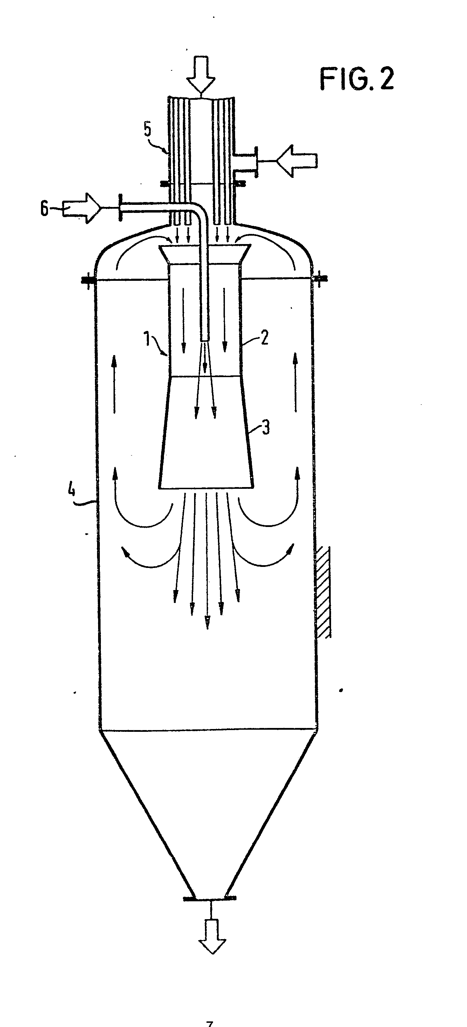

Die Figuren 1 und 2 zeigen die bevorzugten Ausführungsformen des erfindungsgemäßen Verfahrens.

Gemäß Fig. 1 tritt das im Kühler 1 auf die entsprechende Temperatur gekühlte Inertgas mit hoher Geschwindigkeit durch mehrere Einzelrohre 2 in die Abscheidekammer 3 ein. Der Cyanurchloriddampf wird über Leitung 4 in das Zentrum der geteilten Inertgasströme eingeleitet. Nach der Abscheidung verlassen das Inertgas und die Feststoffteilchen die Abscheidekammer über Leitung 5 und werden dem Zyklon 6 zugeführt, in dem eine teilweise Trennung des Gases vom Feststoff in der Weise erfolgt, dass der Hauptanteil des Feststoffs über Leitung 7 ausgetragen wird, während das Inertgas mit einem Restgehalt an feinstem kristallinem Cyanurchlorid über Leitung 8 und den Verdichter 9 dem Kühler 1 wieder zugeführt wird. Zur Reinigung des Inertgases kann über Leitung 10 ein Teilstrom zur Gaswäsche abgezweigt werden.

In Fig. 2 wird eine weitere bevorzugte Verfahrensvariante gezeigt. Das Zentralrohr 1, das aus dem Mischrohr 2 und dem Diffusor 3 besteht, befindet sich am Kopf der Abscheidekammer 4. Das kalte Inertgas tritt mit hoher Geschwindigkeit aus dem Kühler 5 aus und wird in das Mischrohr 2 eingeführt. Es saugt aus dem Ringraum der Abscheidekammer 4 wärmeres Kreisgas an. Nach erfolgter Vermischung beider Ströme wird der heiße Cyanurchloriddampf 6 zugeführt und im Zentrum des Mischgases abgeschreckt. Im Diffusor 3 erfolgt eine Druckerhöhung, die die Zirkulation um das Zentralrohr 1 begünstigt. Der Austrag und die Rückführung des Inertgases erfolgen wie in Fig. 1 dargestellt.

Die nachfolgenden Beispiele erläutern die Erfindung, ohne sie zu beschränken.

Beispiel 1
Es wurde ein Versuch gemäß Fig. 1 durchgeführt, bei dem Cyanurchlorid unter folgenden Bedingungen abgeschieden wurde:

| | |
|---|---|
| Massenstrom an Cyanurchlorid | 12 kg/h |
| Volumenstrom an Inertgas | 1293 kg N/h (1000 m³ N/h) |
| Restgehalt an kristallinem Cyanurchlorid im Inertgas nach der Zentrifugalabscheidung | 450 mg/m³ |
| Korngröße des restlichen Cyanurchlorids im Inertgas (Trennkorngröße des Zyklons) | 5 µm |
| Temperatur des Inertgases am Kühlerausgang | 20°C |
| Geschwindigkeit des Inertgases am Kühlerausgang | 44 m/s |
| Temperatur des Inertgases am Abscheiderausgang | 26°C |

Ergebnis
Die Siebanalyse des erhaltenen Produkts ergab eine Korngrößenverteilung von 98% kleiner als 63 µm. Das Schüttgewicht betrug 260 g/l.
Reinheit des Cyanurchlorids: 98,4 bis 99,2%
Die Verarbeitbarkeit des Cyanurchlorids in Richtung Triazin-Herbizide konnte mit sehr gutem Erfolg nachgewiesen werden.

Beispiel 2
Bei einem Versuch gemäß Fig. 2 wurden folgende Betriebsdaten eingestellt und Ergebnisse erhalten:

| | |
|---|---|
| Massenstrom an Cyanurchlorid | 10,5 kg/h |
| Volumenstrom an Inertgas | 1099 kg N/h (850 m³ N/h) |

Restgehalt an kristallinem
Cyanurchlorid im Inertgas nach der
Zentrifugalabscheidung     510 mg/m³
Korngröße des restlichen
Cyanurchlorids im Inertgas
(Trennkorngröße des Zyklons):     5 µm
Temperatur des Inertgases
am Kühlerausgang     20°C
Temperatur des Inertgases am Ende
der Mischzone     26,5°C
Temperatur des Inertgases
am Abscheiderausgang     27°C

Ergebnis

Die Siebanalyse dieses Produkts ergab folgende Korngrößenverteilung:

> 250 µm   1,06%
125–250 µm   4,91%
63–125 µm   15,56%
<63     µm 78,47%

Das Schüttgewicht betrug 914 g/l.

**Patentansprüche**

1. Verfahren zur Gewinnung von festem Cyanurchlorid aus dem bei der Trimerisierung von Chlorcyan anfallenden Cyanurchloriddampf, dadurch gekennzeichnet, daß man den Cyanurchloriddampf im oberen Teil der Abscheidekammer in das Zentrum vieler einzelner kalter Inertgasströme einleitet, wobei durch ein in die Abscheidekammer eingebautes Zentralrohr eine Vormischung von kaltem Inertgas aus dem Kühler und wärmerem Kreisgas aus der Abscheidekammer bewirkt wird, wonach eine Vermischung dieses Gemisches mit dem heißen Cyanurchloriddampf erfolgt und wobei die Inertgasströme mit einer Temperatur von 0 bis 40°C und einer Geschwindigkeit von 40 bis 80 m/s in einem 50- bis 100fachen Überschuß, bezogen auf die Menge an Cyanurchloriddampf, eingeleitet werden, das feste Cyanurchlorid nach dessen Austritt aus der Abscheidekammer durch Einwirken von Zentrifugal- und Schwerkraft vom Inertgasstrom abtrennt und das Inertgas mit einem Restgehalt von 15 bis 20 Gew.-% an feinstem Cyanurchlorid, bezogen auf das Gesamtgewicht des Cyanurchlorids, nach dessen Abkühlung wieder als Teilströme in die Abscheidekammer zurückführt.

2. Verfahren nach Anspruch 1, dadurch gekennzeichnet, daß man kalte Inertgasströme mit einer Temperatur von 15 bis 30°C einleitet.

3. Verfahren nach Anspruch 1 oder 2, dadurch gekennzeichnet, daß man Inertgas mit einem Restgehalt an feinstem Cyanurchlorid verwendet, dessen Korngröße weniger als 5 µm beträgt.

**Claims**

1. Process for the obtaining of solid cyanuric chloride from the cyanuric chloride vapour obtained by the trimerisation or cyanogen chloride, characterised in that one introduces the cyanuric chloride vapour into the upper part of the separation chamber in the centre of many individual cold inert gas streams, whereby, by means of a central pipe incorporated into the separation chamber, there is brought about a premixing of cold inert gas from the cooler and warmer circulating gas from the separation chamber, whereafter a mixing of this mixture with the hot cyanuric chloride vapour takes place and whereby the inert gas streams with a temperature of from 0 to 40°C and a velocity of 40 to 80 m./sec are introduced in a 50 to 100 fold excess, referred to the amount of cyanuric chloride vapour, the solid cyanuric chloride, after its emergence from the separation chamber, is separated from the inert gas stream by the action of centrifugal and gravitational force and the inert gas with a residual content of 15 to 20 wt.% of very fine cyanuric chloride, referred to the total weight of the cyanuric chloride, is again returned, after its cooling, to the separation chamber.

2. Process according to claim 1, characterised in that one introduces cold inert gas streams with a temperature of 15 to 30°C.

3. Process according to claim 1 or 2, characterised in that one uses inert gas with a residual content of very fine cyanuric chloride, the particle size of which amounts to less than 5 µm.

**Revendications**

1. Procédé de récupération de chlorure de cyanuryle solide à partir de la vapeur de chlorure de cyanuryle produite dans la trimérisation du chlorure de cyanogène, caractérisé en ce qu'on introduit la vapeur de chlorure de cyanuryle dans la partie supérieure de la chambre de séparation au milieu de plusieurs courants individuels de gaz inerte froid, en produisant par un tube central incorporé dans la chambre de séparation un prémélange de gaz inerte froid provenant du refroidisseur et d'un gaz de recyclage plus chaud en provenance de la chambre de séparation, après quoi a lieu un mélange de ce mélange avec la vapeur chaude de chlorure de cyanuryle, en l'occurrence les courants de gaz inerte étant introduits à une température de 0 à 40°C et à une vitesse de 40 à 80 m/seconde en un excès de 50 à 100 fois par rapport à la quantité de vapeur de chlorure de cyanuryle, en ce qu'on sépare le chlorure de cyanuryle solide après sa sortie de la chambre de séparation par l'action d'une force centrifuge et de la pesanteur d'avec le courant de gaz inerte et en ce qu'après son refroidissement on recycle le gaz inerte, qui a une teneur résiduelle de 15 à 20% en poids de chlorure de cyanuryle très fin par rapport au poids total du chlorure de cyanuryle, de nouveau sous la forme de courants partiels dans la chambre de séparation.

2. Procédé selon la revendication 1, caractérisé en ce qu'on introduit des courants de gaz inerte froids à une température de 15 à 30°C.

3. Procédé selon la revendication 1 ou caractérisé en ce qu'on utilise un gaz inerte avec teneur résiduelle en chlorure de cyanuryle très fin, dont la dimension de grain est inférieure à 5 µm.

FIG.1

FIG. 2